# EUROPÄISCHE PATENTANMELDUNG

(11) **EP 1 473 098 A1**
(43) Veröffentlichungstag der Anmeldung: **03.11.2004**
(21) Anmeldenummer: 03101163.8
(22) Anmeldetag: 28.04.2003
(51) Int. Cl.: B21G 1/08, A61B 17/06

(54) **Vorrichtung und Methode zur Crimpung von Nahtmaterial**

(71) Anmelder: SinusPoint AG, 4716 Welschenrohr (CH)
(72) Erfinder: Braun, Peter, 4524 Günsberg (CH); Fink, Heinz, 2740 Moutier (CH); Sattler, Frank, 4514 Lommiswil (CH)
(74) Vertreter: Saam, Christophe

(57) **Zusammenfassung**

Vorrichtung zur automatischen Crimpung von Nahtmaterial mit einem Crimpmodul (4), das zur mindestens teilweise parallelen Crimpung von zwei Nadeln auf einem einzelnen Faden ausgerüstet ist. Methode zur automatischen Crimpung von Nahtmaterial, womit zwei Nadeln mindestens teilweise parallel auf einen einzelnen Faden gecrimpt werden. Die mindestens teilweise parallele Crimpung von zwei Nadel-Faden-Verbindung erlaubt eine gewisse Parallelisierung der Prozess-Schritte und reduziert die Anzahl Manipulierungen der Nadeln und Fäden. Die Produktivität der Crimpvorrichtung ist somit optimal und die Beschädigungsrisiken des Nahtmaterials sind minimiert.

## Beschreibung

Die vorliegende Erfindung betrifft eine Vorrichtung und eine Methode zur Crimpung von Nahtmaterial. Die vorliegende Erfindung betrifft insbesondere eine Methode und eine Vorrichtung zur automatischen Crimpung von Mikro-Nahtmaterial, Kardio-Vaskuläres-Nahtmaterial und/oder Nahtmaterialien ähnlicher Grössen in anderen medizintechnischen Anwendungen.

Das Nahtmaterial welches zum Beispiel in der Chirurgie für das Nähen gebraucht wird, besteht hauptsächlich aus Fäden und Nadeln verschiedenen Typs und Grössen die für unterschiedliche Anwendungen bestimmt sind. Der Durchmesser der Fäden und die Grösse der Nadeln kann unter Umständen sehr klein sein. Bei der Microchirurgie werden zum Beispiel Nadeln mit einer Länge von einigen Millimetern und einem Durchmesser von unter 1 mm gebraucht.

Das Nahtmaterial für medizintechnische Anwendungen wird oft vorkonfektioniert. Fäden einer bestimmten Länge werden im voraus an einem oder beiden Enden mit einer Nadeln verbunden. Dann wird das Material sterilisiert und eingepackt.

Die Vorkonfektionierung dieses Nahtmaterials ist meistens aufwendig und teuer. Im Allgemeinen wird die Faden-Nadel-Verbindung manuell gefertigt. Ein Ende des Fadens muss genau in eine Öffnung oder in einen Kanal der Nadeln eingeführt werden, das dann mit einem Crimpwerkzeug auf die Nadel gecrimpt bzw. geleimt wird.

Vorrichtungen zur Automatisierung dieses Arbeitsvorgangs sind wohl bekannt. Sie sind aber meistens nicht geeignet für kleines Nahtmaterial, insbesondere sind sie nicht an Mikro-Nahtmaterial adaptiert.

Bekannte automatische Vorrichtungen sind auch nicht für die vollautomatische Vorkonfektionierung von Nahtmaterial mit zwei Nadeln an einem einzelnen Faden geeignet. Im Stande der Technik muss jeder Faden zwei aufeinander folgende Mal durch die gleiche Vorrichtung oder durch zwei selbständige Vorrichtungen bearbeitet werden. Der Faden wird zum Beispiel in einer ersten Richtung zur Crimpung der ersten Nadel-Faden-Verbindung in eine automatische Vorrichtung eingeführt, dann wird er von Hand gedreht und wieder in die gleiche oder in eine andere Vorrichtung zur Crimpung der zweiten Nadel-Faden-Verbindung eingeführt. Der Aufwand und die Fehlerwahrscheinlichkeit werden also durch dieses Vorgehen wesentlich erhöht. Die Kapazität der Vorrichtung bei der Herstellung von solchem Nahtmaterial wird auch ungefähr um die Hälfte reduziert, verglichen mit der Herstellung von Nahtmaterial mit nur einer Nadel pro Faden.

Ein Ziel der Erfindung ist also, eine Vorrichtung und eine Methode vorzuschlagen, die für die automatische Herstellung von Nahtmaterial mit zwei Nadeln pro Faden besonders geeignet sind.

Ein weiteres Ziel der Erfindung ist, eine Vorrichtung und eine Methode vorzuschlagen, die eine erhöhte Durchsatzrate gegenüber Vorrichtungen und Methoden des Standes der Technik aufweist.

Diese Ziele werden durch eine Vorrichtung und eine Methode erreicht, welche die Merkmale des entsprechenden unabhängigen Anspruchs aufweisen.

Insbesondere werden diese Ziele durch eine Vorrichtung zur automatischen Crimpung von Nahtmaterial mit einem Crimpmodul, das für die mindestens teilweise parallele Crimpung von zwei Nadeln auf einen einzelnen Faden ausgerüstet ist, erreicht. Diese Ziele werden auch durch eine Methode zur automatischen Crimpung von Nahtmaterial erreicht, wobei zwei Nadeln mindestens teilweise parallel auf einen einzelnen Faden gecrimpt werden. Die mindestens teilweise parallele Crimpung von zwei Nadel-Faden-Verbindungen erlaubt eine gewisse Parallelisierung der Prozessschritte und reduziert die Anzahl Manipulierungen der Nadeln und Fäden. Die Produktivität der Crimpvorrichtung ist somit optimal und die Beschädigungsrisiken des Nahtmaterials sind minimiert.

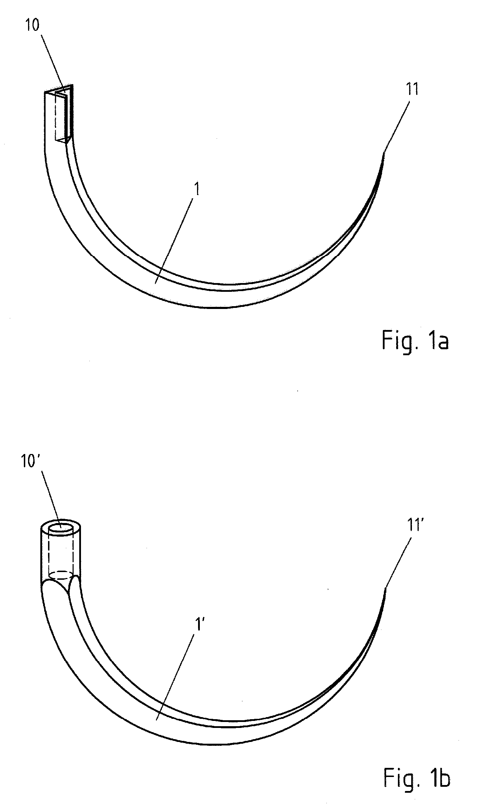

Die vorliegende Erfindung wird anhand der Beschreibung einer bevorzugten Ausführungsform und mit Hilfe der Figuren 1a bis 5 näher erläutert, wobei
die Figuren 1a und 1b je eine Nadel mit verschiedenen Typen von Fadenaufnahmen zeigen,
die Figur 2 eine Perspektivansicht der Vorrichtung gemäss der Erfindung ist,
die Figur 3 den Nadelgreifer des Nadelroboters zeigt,
die Figur 4 den Fadengreifer des Fadenroboters zeigt,
die Figur 5 ein Beispiel von fertigem vorkonfektionierten Nahtmaterial illustriert.

In einer bevorzugten Ausführungsform dient die Vorrichtung der Erfindung der automatischen Crimpung von Mikro-Nahtmaterial und/oder von Kardio-Vaskuläres-Nahtmaterial. Die Nadeln haben dann typischerweise einen Durchmesser von 0.03 bis 0.5 mm und eine Länge von 3 bis 25 mm. Sie sind üblicherweise in einem Winkel von 0° bis 180° gebogen. Diese Abmessungen werden als illustratives aber in keinem Fall limitatives Beispiel angegeben. Der Fachmann wird leicht einsehen, dass die Methode und die Vorrichtung der Erfindung auch zur Crimpung von Nahtmaterial mit kleineren oder grösseren Abmessungen eingesetzt werden können. Die Nadeln 1, 1' haben an einer Seite eine Spitze 11, 11' mit einem bestimmten Profil, währenddessen auf der anderen Seite eine Fadenaufnahme 10, 10' zur Einführung eines Fadens vorgesehen ist. Die Fadenaufnahme 10, 10' ist meistens entweder eine V-förmige Rinne 10 am Ende der Nadel 1 entlang ihre Längsachse, oder ein Loch 10' im Durchmesser der Nadel 1' ebenfalls entlang ihrer Längsachse. Im Rahmen der Erfindungen können die Fäden aus allen in der medizintechnischen Anwendung verwendeten Materialien wie zum Beispiel Nylon-, Polypropylen-, Polyester-, Seidefäden, usw. sein.

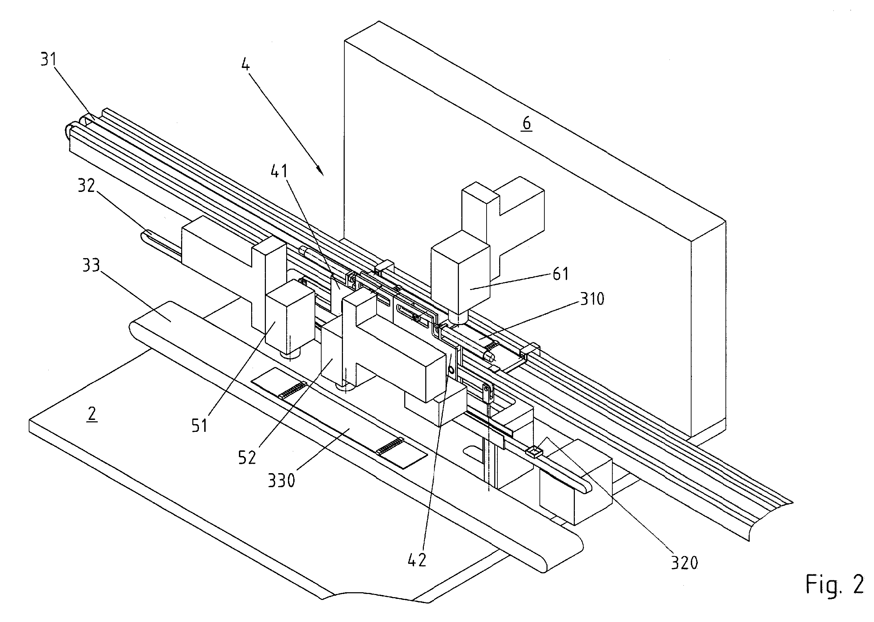

Die automatische Crimpvorrichtung, die in einer bevorzugten Ausführungsform in der Figur 2 dargestellt ist, weist vorzugsweise eine flache Stützfläche zum Beispiel in der Form eines metallischen Tisches oder eines Steintisches 2 auf. Die Tischplatte ist zum Beispiel mit nicht dargestellten Bohrungen und Aussparungen zur Ausrichtung und/oder Befestigung sämtlicher Elemente der Vorrichtung überarbeitet.

Die Crimpvorrichtung umfasst ein erstes automatisches Transportsystem 31 zur Fadenzuführung und ein zweites automatisches Transportsystem 32 zur Nadelzuführung. Diese Transportsysteme sind zum Beispiel zwei parallele Förderbänder 31, 32. Die Nadeln und die Fäden werden vorzugsweise in Nadel- bzw. Fadenträgern 310, 320 auf dem entsprechenden Förderband 31, 32 transportiert. Ein drittes Transportsystem zum Beispiel ein drittes paralleles Förderband 33 dient zur Abführung der auf Endproduktträger 330 gelegten fertigen Nahtmaterialen.

Zwischen dem Fadentransportband 31 und dem Nadeltransportsystem 32 befindet sich ein Crimpmodul 4 zur automatischen Herstellung der Faden-Nadel-Verbindungen. Das Crimpmodul 4 ist mit zwei motorisierten Crimpstationen 41, 42 ausgerüstet. Die nicht dargestellten Backen der Crimpstationen 41, 42 sind dem Typ und der Grösse der Nadeln angepasst. Sie können vorzugsweise ausgewechselt werden, ohne dass die Crimpstation 41 oder 42 aus der automatischen Crimpvorrichtung demontiert werden muss. Damit kann die Vorrichtung einfach an die Crimpung von Nadel-Faden-Verbindungen verschiedenen Typs und/oder Grössen angepasst werden.

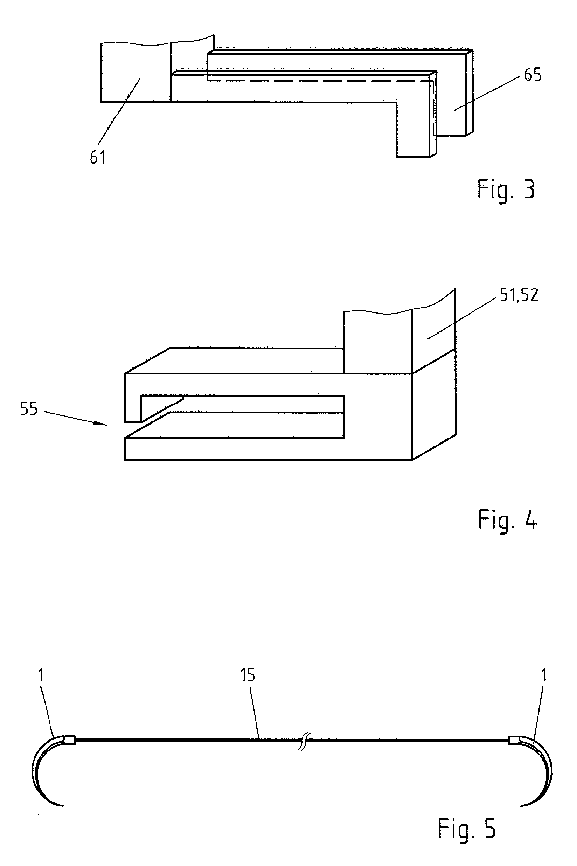

Jeder Crimpstation 41, 42 ist ein Nadelroboter 51, 52 zugeordnet. Die Nadelroboter 51, 52 stehen vorzugsweise zwischen dem Nadeltransportsystem 32 und dem Endprodukttransportsystem 33 gegenüber dem Crimpmodul 4. Die Nadelroboter 51, 52 dienen der Entnahme der Nadeln aus den Nadelträgern 320 und ihrer Positionierung im Crimpmodul 4, sowie der anschliessenden Ablage der fertigen Nahtmaterialen auf dem Endproduktträger 330. An jedem Nadelroboter 51, 52 ist ein Nadelgreifer installiert (Figur 4), der zum Beispiel aus einer Zange 55 besteht, deren Klemmkraft so definiert ist, dass diese bei der Klemmung der Nadeln, keine Deformation bzw. Beschädigung der Nadeln hervorruft.

Auf der anderen Seite des Crimpmoduls 4 steht ein Fadenroboter 6 zur Entnahme der Fäden aus den Fadenträgern 310 und ihrer Positionierung im Crimpmodul 4, die mit einem Fadengreifer 65 in Form einer Zange ausgerüstet ist (Figur 3). Um eine Deformation bzw. Verletzung der Fäden zu vermeiden, wird die Klemmkraft des Fadengreifers 65 vorzugsweise gegen Null gewählt. Aus demselben Grund sind auch die Klemmbacken vorzugsweise aus einem flexiblen Material.

Ein nicht dargestelltes optisches Kontrollsystem dient zur Überwachung des Vorganges. Das optische Kontrollsystem umfasst vorzugsweise bei jeder Crimpstation 41, 42 drei Kameras. Zwei vertikal orientierte Kameras, die je an einer Seite der entsprechenden Crimpstation 41 oder 42 platziert sind, dienen zur Bestimmung und zur Korrektur der Nadel- bzw. der Fadenpositionierung auf der horizontalen Ebene kurz vor der Einführung der Nadel bzw. des Fadens in die entsprechende Crimpstation 41 oder 42. Eine horizontal orientierte Kamera dient zur Positionserfassung der Nadel in der vertikalen Achse und der Positionierung des Fadens in der Fadenaufnahme. In einer anderen Ausführungsform dient die horizontal orientierte Kamera auch weiter zur optischen Qualitätskontrolle der fertigen Crimpung.

Aus Platzgründen sind die Kameras vorzugsweise unter dem Tisch 2 befestigt und die Objektive nehmen die Bilder durch ein Loch in der Tischplatte auf. Die Visionsachse der dritten Kamera ist dann vorzugsweise mittels einem Prisma auf dem Tisch 2 horizontal orientiert.

Die Crimpvorrichtung wird durch ein nicht dargestelltes Steuersystem gesteuert. Das Steuersystem ist zum Beispiel auf einem Rechner implementiert, der durch Kommunikations- und/oder Steuerleitungen mit dem optischen Kontrollsystem und den motorisierten Elementen der Crimpvorrichtung verbunden ist. Das Steuersystem analysiert insbesondere die Daten des optischen Kontrollsystems und sendet dem Fadenroboter 6, den Nadelrobotern 51, 52 und den Crimpstationen 41, 42 die notwendigen Befehle zum korrekten Funktionieren der Crimpvorrichtung.

Wenn die im voraus auf eine bestimmte Länge zugeschnittenen Fäden der Crimpvorrichtung zugeführt werden, liegen diese auf einem Fadenträger 310. Der Fadenträger 310 umfasst vorzugsweise einen stabiles Trägermedium, auf welchem ein weiches Trägermedium installiert ist. Im weichen Trägermedium befinden sich zwei voneinander entfernte identische Reihen von Schlitzen. Die Fäden werden auf den Fadenträger 310 so positioniert, dass sie alle zwischen den zwei Reihen von Schlitzen gleich orientiert sind. Der mit Fäden bestückte Fadenträger 310 wird mittels des Fadentransportsystems 31 aus einem nicht dargestellten Magazin bis vor das Crimpmodul 4 geführt. Dort wird er vorzugsweise in einer vordefinierten Lage mit nicht dargestellten mechanischen Mitteln temporär fixiert, damit die Position der auf dem Fadenträger 310 vorhandenen Fäden bekannt und stabil ist.

Die Nadeln werden der Crimpvorrichtung auf einem Nadelträger 320 mittels dem Nadeltransportsystem zugeführt. Der Nadelträger besteht zum Beispiel aus einer stabilen Trägerfläche, die mit einer weichen Materie zum Schutz der Nadeln abgedeckt ist, auf welcher die Nadeln lageorientiert vorpositioniert sind. Der Nadelträger 320 wird bis vor das Crimpmodul 4 transportiert und dort mit ebenfalls nicht dargestellten mechanischen Mitteln in einer vordefinierten Lage temporär fixiert, damit die Position der auf dem Nadelträger 320 vorhandenen Nadeln bekannt und stabil ist.

Mit dem Endprodukttransportsystem 33 wird ein leerer Endproduktträger 330 ebenfalls vor das Crimpmodul 4 geführt und dort in einer vordefinierten Lage temporär fixiert. Der Endproduktträger 330 ist zum Beispiel identisch mit dem Fadenträger 310.

Die Faden-, Nadel- und Endproduktträger 310, 320, 330 werden hier oben anhand von illustrativen aber in keinem Fall limitativen Beispielen beschrieben. Der Fachmann wird leicht einsehen, dass diese Träger auch anders ausgeführt werden können. Sie müssen aber mit dem gewählten Transportsystem kompatibel sein, und die Position und Orientierung der getragenen Elemente muss vorzugsweise mit einer gewissen Genauigkeit gehalten werden.

Die automatische Crimpvorrichtung der Erfindung ist dank dem Crimpmodul 4 mit zwei Crimpstationen 41, 42 zur Herstellung von Nahtmaterialen mit zwei Nadeln 1 pro Faden 15 (Figur 5) besonders geeignet, da die Crimpvorgänge der beiden Nadel-Faden-Verbindungen eines gleichen Nahtmaterials mindestens teilweise parallel stattfinden können. Dieses Nahtmaterial wird mit dem folgenden Vorgang hergestellt.

Der erste Nadelroboter 51 entnimmt dem temporär fixierten Nadelträger 320 eine Nadel und positioniert sie in der ersten Crimpstation 41. Der Nadelroboter 51 hält die Nadel so, damit die Fadenaufnahme frei bleibt und gegen den Fadenroboter 6 orientiert ist. Kurz bevor die Nadel in die Crimpstation 41 eingeführt wird, wird sie im Fokus einer nicht dargestellten vertikal orientierten Kamera des optischen Kontrollsystems gehalten. Das von der Kamera aufgenommene Bild wird durch das Steuersystem analysiert, um die genaue horizontale Positionierung der Nadel zu bestimmen. Gegebenfalls wird die Lage der Nadel korrigiert, indem das Steuersystem Befehle zur Bewegung des ersten Nadelroboters 51 sendet. Danach wird die Nadel von dieser bekannten Lage um einen bestimmten Weg bewegt, damit sie sich in der Crimpstation 41 befindet. Die vertikale Lage der Nadel in der Crimpstation 41 wird durch das Steuersystem dank der Informationen von der nicht dargestellten horizontal orientierten Kamera bestimmt und korrigiert.

Gleichzeitig führt der Fadenroboter 6 ein Ende des Fadens in das erste Crimpstation 41. Kurz vor der ersten Crimpstation 41 wird der Faden im Fokus der anderen nicht dargestellten vertikal orientierten Kamera des optischen Kontrollsystems gehalten. Das von der Kamera aufgenommene Bild wird durch das Steuersystem analysiert, um die genaue horizontale Positionierung des Fadens zu bestimmen. Gegebenfalls wird die Lage des Fadens korrigiert, indem das Steuersystem Befehle zur Bewegung des Fadenroboters 6 sendet. Danach wird der Faden von dieser bekannten Lage um einen bestimmten Weg bewegt, und in der Fadenaufnahme der Nadel positioniert. Die genaue Positionierung des Fadens in der Fadenaufnahme wird mittels der nicht dargestellten horizontal orientierten Kamera bestimmt und korrigiert. Wenn der Faden in der Nadel korrekt positioniert ist wird vom Steuersystem der Befehl erfassen, die Crimpstation 41 zu aktivieren. Die erste Nadel-Faden-Verbindung wird somit durch Crimpung der Nadelaufnahme um den Faden herum hergestellt.

Nach dem Wiederöffnen der Crimpstation 41 wird gemäss einer weiteren Ausführungsform der Erfindung eine optisch qualitative Überprüfung der Nadel-Faden-Verbindung mit dem optischen Kontrollsystem durchgeführt. Die von den Kameras aufgenommenen Bilder werden zum Beispiel auf nicht dargestellten Bildschirmen dargestellt, um eine menschliche Überprüfung zu erlauben, und/oder durch das Steuersystem analysiert, um die Qualität der Crimpung anhand von vorbestimmten Kriterien automatisch zu bestimmen.

Kurz nach dem Schliessen der ersten Crimpstation 41 entnimmt der zweite Nadelroboter 52 dem Nadelträger 320 ebenfalls eine Nadel und positioniert sie in der zweiten Crimpstation 42, nachdem Ihre Lage dank dem entsprechenden horizontal orientierten Kamera kontrolliert und gegebenenfalls korrigiert wurde. Der Fadenroboter 6 löst gleichzeitig das erste Ende des Fadens, entnimmt dem Fadenträger 310 das zweite Ende des gleichen Fadens, kontrolliert und korrigiert seine Lage mittels dem optischen System und positioniert es in der Fadenaufnahme der durch den zweiten Nadelroboter 52 positionierten Nadel. Die Positionierung des Fadens in der Fadenaufnahme wird wie zuvor dank der Information aus einer vertikal orientierten Kamera des optischen Kontrollsystems bestimmt und mit durch das Steuersystem angetriebenen Bewegungen des Fadenroboters 6 korrigiert. Wenn der Faden in der Fadenaufnahme korrekt positioniert ist wird durch das Steuersystem ein Befehl zur Bewegung der zweiten Crimpstation 42 ausgelöst.

In einer weiteren Ausführungsform der Erfindung wird die Qualität der zweiten Nadel-Faden-Verbindung wie bei der ersten Nadel-Faden-Verbindung mittels des optischen Kontrollsystems überprüft.

Das fertige Nahtmaterial wird dann durch den ersten und den zweiten Nadelroboter 51, 52 auf den Endproduktträger 330 gelegt. Beide Enden des Fadens werden vorzugsweise in sich gegenüberliegenden Schlitzen eingelegt, damit das fertige Nahtmaterial zwischen den beiden Schlitzesreihen positioniert ist.

Die oben beschriebenen Prozess-Schritte werden mit den nächsten in den entsprechenden Trägern 310, 320 vorhandenen Fäden und Nadeln zur Fertigung von weiteren Nahtmaterialen wiederholt.

Nachdem der letzte Faden aus dem Fadenträger 310 entnommen wird, wird der Fadenträger 310 mechanisch endfixiert und mit dem Fadentransportsystem 31 zum Beispiel in ein nicht dargestelltes Magazin weiterbefördert. Gleichzeitig wird ein neuer voller Fadenträger 310 vor das Crimpmodul 4 geführt und dort fixiert. Gleicherweise wird der Nadelträger 320 nach der Entnahme der letzten Nadel endfixiert und mit dem Nadeltransportsystem 32 zum Beispiel in ein nicht dargestelltes Magazin weiterbefördert, während ein neuer voller Nadelträger 320 vor das Crimpmodul 4 geführt und dort fixiert wird.

Gemäss der oben beschriebenen Ausführungsform der Erfindung, werden die Nadeln durch beide Nadelroboter 51, 52 aus dem gleichen Nadelträger 320 entnommen. In einer weiteren Ausführungsform sind mindestens zwei Nadelträger vor dem Crimpmodul 4 positioniert, wobei jeder Nadelroboter 51, 52 Nadeln aus einem verschiedenen Nadelträger entnimmt. Diese Ausführungsform hat gegenüber der Vorherigen den Vorteil, dass die Bewegungen der Nadelroboter 51, 52 reduziert werden können, indem ein Nadelträger zwischen jedem Nadelroboter 51 oder 52 und der entsprechenden Crimpstation 41 oder 42 positioniert wird. Gemäss dieser weiteren Ausführungsform wird jeder Nadelträger zuerst vor einem Nadelroboter positioniert, bis dieser die Hälfte der Nadeln entnommen hat. Dann wird der Nadelträger dem Nadelroboter zugeführt, und durch einen neuen vollen Nadelträger ersetzt. In einer noch weiteren Ausführungsform umfasst das Nadeltransportsystem zwei parallele Förderbänder. Dann kann die Nadelzuführung für beide Nadelroboter unabhängig laufen.

Wie oben schon erwähnt ist die automatische Crimpvorrichtung gemäss einer bevorzugten Ausführungsform der Erfindung zur Herstellung von Mikro-Nahtmaterial geeignet. Die korrekte Positionierung der Nadel in der Crimpstation und des Fadens in den Nadelkanälen ist wegen der Dimensionen der entsprechenden Elemente ein Vorgang, der eine sehr hohe Arbeitsgenauigkeit erfordert. Die Analyse der Bilder aus dem optischen Kontrollsystem und die Steuerung der Faden- und Nadelroboter 6, 51, 52 müssen also sehr präzise sein. Die Präzision des Fadenroboters 6 ist in diesem Sinne besonders wichtig. Deshalb ist dieser Roboter ein sehr wichtiges und teures Element der Crimpvorrichtung.

Dank dem oben beschriebenen Vorgang zur Crimpung von Nahtmaterial mit zwei Nadel pro Faden wird der gleiche Fadenroboter 6 zur Positionierung beider Enden des Fadens benützt, was eine wesentliche Begrenzung der Kosten für die Crimpvorrichtung der Erfindung erlaubt. Ein weiterer Vorteil des oben beschriebenen Vorgangs ist, dass die Bewegungen der Roboter zur Positionierung der Elemente der ersten Faden-Nadel-Verbindung die Positionierung der Elemente der zweiten Verbindung nicht stören kann, da die Vorgänge der Fadenpositionierung nicht gleichzeitig ablaufen.

Die Benützung von zwei parallelen durch das gleiche Steuersystem koordinierten Crimpstationen 41, 42 erlaubt jedoch eine gewisse Parallelisierung der Prozess-Schritte und reduziert die Anzahl Manipulierungen der Nadeln und Fäden. Die Produktivität der Crimpvorrichtung ist somit optimal und die Beschädigungsrisiken des Nahtmaterials sind minimiert.

Bei der Konfektionierung von grösseren Nahtmaterialen, die eine weniger präzise Positionierung der Elemente erfordern, oder bei der Benutzung von stabileren und also auch teueren Robotern und/oder Crimpstationen wäre es aber im Rahmen der Erfindung denkbar, beide Positionierungs- und Crimpungsschritte gleichzeitig durchzuführen. Die Crimpvorrichtung weist dann vorzugsweise zwei voneinander unabhängige Fadenroboter oder einen Fadenroboter mit zwei Fadengreifern auf. Gemäss dieser Ausführungsform der Erfindung können also zwei Crimpvorgänge völlig parallel ablaufen. Beide Nadelroboter entnehmen gleichzeitig je eine Nadel und positionieren sie in der entsprechenden Crimpstation. Die zwei Fadengreifer, die entweder auf einem einzigen Fadenroboter oder auf zwei voneinander unabhängige Fadenroboter installiert sind, entnehmen gleichzeitig je das Ende eines Fadens und positionieren es in der Fadenaufnahme der entsprechenden Nadel. Eine solche Crimpvorrichtung ist wohl zur parallelen Crimpung von zwei Faden-Nadel-Verbindungen auf einem einzigen Faden geeignet. Sie kann aber auch zur parallelen Crimpung von zwei Faden-Nadel-Verbindungen auf zwei verschiedenen Faden benützt werden, wobei ihre Kapazität das doppelte der Kapazität einer Crimpvorrichtung mit nur einer Crimpstation ist.

In einer anderen Ausführungsform der Erfindung umfasst die Crimpstation eine nicht dargestellte Teststation zur mechanischen Qualitätsprüfung der hergestellten Nadel-Faden-Verbindungen. Die Teststation besteht zum Beispiel aus einer Prüfzange, indem mit einer vorbestimmten Zugkraft am Faden gezogen wird, währenddem die Nadel gegen einen Anschlag gehalten wird. Gemäss dieser Ausführungsform der Erfindung wird nach der Crimpung die erste Nadel-Faden-Verbindung durch den ersten Nadelroboter zur Prüfzange gebracht. Der Faden wird in einen Fadenschlitz im Anschlag eingeführt. Die Nadel wird unter einer vorbestimmten Spannung bis zum Anschlag über den Faden gezogen. Nach Erreichen einer vorbestimmten Prüfkraft wird ein Signal an den Nadelroboter geschickt, dass die geprüfte Nadel-Faden-Verbindung in Ordnung ist. Die Zugkraft wird reduziert. Der Nadelroboter ergreift die Nadel wieder, entnimmt sie aus der Teststation und bringt sie zum Endproduktträger. Falls die Crimpung während dem Test zerstört wird, wird durch das Steuersystem ein "nicht in Ordnung" Signal gegeben. Das defekte Nahtmaterial wird dann zum Beispiel mittels Unterdruck abgesaugt.

Wenn der Faden mit zwei Nadeln bestückt ist, wird die zweite Nadel mittels dem zweiten Nadelroboter zur Teststation gebracht und mit der Prüfzange getestet. Wenn beide Nadel-Faden-Verbindung in Ordnung sind, wird das fertig konfektionierte Nahtmaterial auf den Endproduktträger orientiert gelegt.

Wenn die Aufnahmekapazität des Endproduktträgers 330 erreicht ist, wird durch das Steuersystem der mechanischen Mittel ein Signal zur temporären Fixierung geschickt, dass diese gelöst werden kann. Der bestückte Endproduktträger 330 wird durch das Endprodukttransportsystem 33 zum Beispiel in ein nicht dargestelltes Magazin geführt, währenddessen der Crimpvorrichtung ein leerer Endproduktträger 330 zugeführt wird.

In einer weiteren Ausführungsform umfasst die automatische Crimpvorrichtung der Erfindung zwei Teststationen, damit beide Nadel-Faden-Verbindungen gleichzeitig getestet werden können.

Die automatische Crimpvorrichtung der Erfindung ist dank dem Crimpmodul 4 mit zwei Crimpwerkzeugen 41, 42 zur Herstellung von Nahtmaterialen mit zwei Nadeln pro Faden besonders geeignet. Der Fachmann wird aber leicht einsehen, dass die gleiche Vorrichtung auch zur Konfektionierung von Nahtmaterial mit nur einer Nadel pro Faden benutzt werden kann, indem zum Beispiel nur eine Crimpstation benützt wird, oder indem zwei Nahtmaterialen parallel hergestellt werden.

Wie früher schon erwähnt werden die Bewegungen der Fadenroboter 6, die Fadenroboter 51, 52, die Crimpstationen 41, 42 und die Transportsysteme 31, 32, 33 mittels einem Steuersystem gesteuert und kontrolliert. Alle Prozess-Schritte der Crimpung sind also für eine optimale Produktivität koordiniert und die Herstellung des Nahtmaterials läuft völlig automatisch.

Das Steuersystem ist zum Beispiel so aufgebaut, dass alle Prozessschritte einzeln gesteuert werden können und somit eine flexible automatische oder teilautomatische Produktion möglich ist. Das Steuersystem erlaubt zum Beispiel die Steuerung der Crimpvorrichtung so, dass nur eine Crimpstation benützt wird, dass Nahtmaterialen mit einer oder mit zwei Nadeln produziert werden und/oder dass den mechanischen Test auf einen Teil der Produktion oder auf die ganze Produktion nicht durchgeführt wird, usw. Unter Umständen kann vorzugsweise das Steuersystem so konfiguriert werden, dass zum Beispiel die Zuführung und/oder die Entnahme der Nadel-, Faden- und/oder Endproduktträger manuell stattfindet.

## Patentansprüche

1. Vorrichtung zur automatischen Crimpung von Nahtmaterial, mit einem Crimpmodul (4), **dadurch gekennzeichnet, dass** besagtes Crimpmodul (4) zur mindestens teilweise parallelen Crimpung von zwei Nadeln auf einen einzelnen Faden ausgerüstet ist.

2. Crimpvorrichtung gemäss dem vorherigen Anspruch, wobei das besagte Nahtmaterial medizintechnisches Mikro-Nahtmaterial und/oder Kardio-Vaskuläres-Nahtmaterial ist.

3. Crimpvorrichtung gemäss einem der vorherigen Ansprüche, wobei besagte Nadeln einen Durchmesser von 0.03 bis 0.5 mm und eine Länge von 3 bis 25 mm haben.

4. Crimpvorrichtung gemäss einem der vorherigen Ansprüche, wobei besagtes Crimpmodul (4) zwei Crimpstationen (41, 42) enthält.

5. Crimpvorrichtung gemäss einem der vorherigen Ansprüche, die ein optisches Kontrollsystem zur Bestimmung der Lageposition des Fadens und/oder der Nadel im besagten Crimpmodul (4) enthält.

6. Vorrichtung gemäss einem der vorherigen Ansprüche, die ein Fadentransportsystem (31) zur automatischen Zuführung von Fäden zum besagten Crimpmodul (4) umfasst.

7. Vorrichtung gemäss dem vorherigen Anspruch, wobei das besagte Fadentransportsystem ein Förderband (31) ist.

8. Vorrichtung gemäss einem der vorherigen Ansprüche, die ein Nadeltransportsystem (32) zur automatischen Zuführung von Nadeln zum besagten Crimpmodul (4) umfasst.

9. Vorrichtung gemäss dem vorherigen Anspruch, wobei das besagte Nadeltransportsystem ein Förderband (32) ist.

10. Vorrichtung gemäss einem der vorherigen Ansprüche, die ein Endprodukttransportsystem (33) zur automatischen Abführung von fertigem Nahtmaterial ab dem besagten Crimpmodul (4) umfasst.

11. Vorrichtung gemäss dem vorherigen Anspruch, wobei das besagte Endprodukttransportsystem ein Förderband (33) ist.

12. Vorrichtung gemäss einem der vorherigen Ansprüche, die einen Fadenroboter (6) zur Entnahme von Fäden und deren Positionierung im besagten Crimpmodul (4) umfasst.

13. Vorrichtung gemäss dem vorherigen Anspruch, wobei der besagte Fadenroboter (6) einen beweglichen Arm (61) zur Positionierung beider Enden der besagten Fäden in das besagte Crimpmodul (4) umfasst.

14. Vorrichtung gemäss einem der Ansprüche12 oder 13, wobei der besagte Fadenroboter (6) mit einem Fadengreifer (65) in Form einer Zange ausgerüstet ist.

15. Vorrichtung gemäss einem der vorherigen Ansprüche, die mindestens einen Nadelroboter (51, 52) zur Entnahme von Nadeln und deren Positionierung in die besagte Crimpstation (4) umfasst.

16. Vorrichtung gemäss dem vorherigen Anspruch, wobei der besagte mindestens eine Nadelroboter (51, 52) mit einem Nadelgreifer (55) in Form einer Zange ausgerüstet ist.

17. Vorrichtung gemäss einem der vorherigen Ansprüche, mit mindestens einer Teststation zur mechanischen Prüfung der Crimpung.

18. Methode zur automatischen Crimpung von Nahtmaterial, **dadurch gekennzeichnet, dass** zwei Nadeln mindestens teilweise parallel an einem einzelnen Faden gecrimpt werden.

19. Methode gemäss dem vorherigen Anspruch, die folgende Schritte umfasst:
Positionierung einer ersten Nadel in einer ersten Crimpstation (41 ),
Positionierung eines Fadens in der Fadenaufnahme der besagten ersten Nadel,
Crimpung der Fadenaufnahme der ersten Nadel auf den besagten Faden mit der besagten ersten Crimpstation (41) zur Herstellung einer ersten Nadel-Faden-Verbindung,
Positionierung einer zweiten Nadel in einer zweiten Crimpstation (42),
Positionierung des besagten Fadens in der Fadenaufnahme der besagten zweiten Nadel,
Crimpung des Kanals der ersten Nadel auf den besagten zweiten Faden mit der besagten zweiten Crimpstation (41) zur Herstellung einer zweiten Nadel-Faden-Verbindung.

20. Methode gemäss dem vorherigen Anspruch mit dem folgenden zusätzlichen Schritt:
mechanische Prüfung der besagten ersten und zweiten Nadel-Faden-Verbindungen.
